# EUROPEAN PATENT APPLICATION

(11) **EP 4 173 621 A1**
(43) Date of publication of application: **03.05.2023**
(21) Application number: 21306521.2
(22) Date of filing: 29.10.2021
(51) Int. Cl.: A61K 31/4425, A61P 11/00, A61P 31/14

(54) **PHARMACEUTICAL COMPOSITION, ITS USE AS A DRUG AND NEW COMPOUNDS, ESPECIALLY FOR TREATING SARS-COV-2 INFECTION**

(71) Applicant: Université de Bordeaux, 33000 Bordeaux (FR); Centre National de la Recherche Scientifique, 75016 Paris (FR)
(72) Inventor: PARISSI, Vincent, 33300 Bordeaux (FR); SOUSA, Sergio, 4440-752 Valongo (PT); LAPAILLERIE, Delphine, 33600 Pessac (FR); DELELIS, Olivier, 78220 Viroflay (FR); MEERTENS, Laurent, 95170 Deuil La Barre (FR); GALLOIS-MONTBRUN, Sarah, 92370 Chaville (FR); TEULADE-FICHOU, Marie-Paule, 75014 Paris (FR); LARTIA, Rémy, 38610 Gières (FR); BORDEAU, Guillaume, 31000 Toulouse (FR)
(74) Representative: Novagraaf Technologies

(57) **Abstract**

The present invention relates to a pharmaceutical composition comprising a compound having formula (I) below: 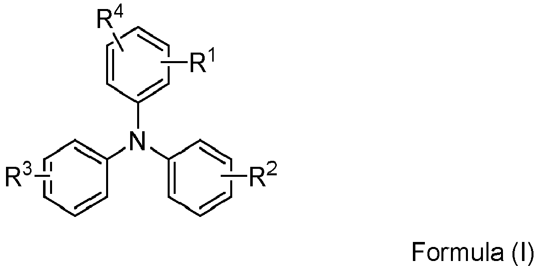 wherein:
- R¹ is chosen from the group comprising: 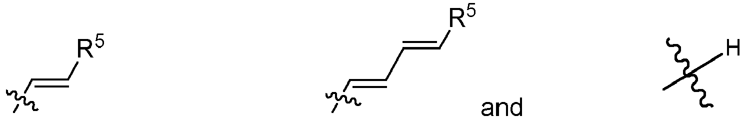 - R² is chosen from the group comprising: 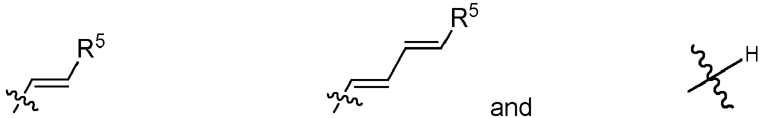 - R³ is chosen from the group comprising: 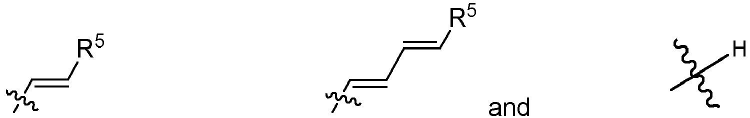 with R⁵ being chosen from the group comprising a heterocyclic compound having 5 or 6 members and a heterocycle compound having 5 or 6 members condensed with a benzene ring,
- R⁴ is chosen from the group comprising a hydrogen, an alkyl or a PEG chain, terminated by a function chosen among OH, NH₂ and COOH, and its use as a medicament.

## Description

### Technical field

The present invention refers to a pharmaceutical composition comprising a compound, and their use as a medicament, especially for use in the treatment of an infection.

Therefore, the present invention has utility in medical field, especially in the field of treatment of infection diseases.

In the description below, the references into brackets ([ ]) refer to the listing of references situated at the end of the text.

### Background of the Invention

Severe Acute Respiratory Syndrome-coronavirus 2 (SARS-CoV-2) is the positive-strand RNA virus causing agent of severe respiratory syndrome in humans (COVID-19).

Viral entry into cells requires the interaction between the spike S protein of the virus and the Angiotensin-Converting enzyme 2 ACE2 at the surface of the cell membrane. ACE2 has been reported as receptor for SARS-CoV-2 in lung as previously shown for SARS-CoV responsible for the first SARS outbreak in 2003. ACE2 is expressed in human airway epithelia, lung parenchyma, vascular endothelia, kidney cells, and small intestine cells but only weakly detected in the brain. Thus, converging data from the literature indicate the S/ACE2 interaction remains the central entry way for SARS-CoV-2 virus. Being the first contact point between the virus and the cell, the S/ACE2 complex constitute an attractive target for blocking the very early events of the viral infection.

While several vaccines have been developed since the beginning of the pandemic, no efficient drugs have been identified as having strong effect on viral replication usable in clinic, partly due to the lack of understanding of viral replication processes.

Therefore, a better understanding of the replication processes and the search for new antiviral approaches remain crucial.

Thus, a need exists of drugs allowing to treat, or at least decrease the symptoms due to SARS-CoV-2 infection. The present invention fulfills these and other needs.

### Description of the invention

The Applicant surprisingly found that compounds derived from triphenylamine have the capability to block the infectivity of lentiviral vectors pseudotyped with the SARS-CoV-2 S protein as well as wild type and variants SARS-CoV-2 circulating strains in various human cell lines, including pulmonary cells naturally susceptible to the infection.

AlphaLISA and biolayer interferometry confirmed a direct inhibitory effect of the compounds on the S/ACE2 association.

Thus, after extensive works, the Applicant found novel drugs capable to specifically block the SARS-CoV-2 entry step by impairing the ACE2/S interaction leading to inhibition of the viral SARS-CoV-2 replication in naturally susceptible cells.

Accordingly, in a first aspect, the present invention provides a pharmaceutical composition comprising a compound having formula (I) below: 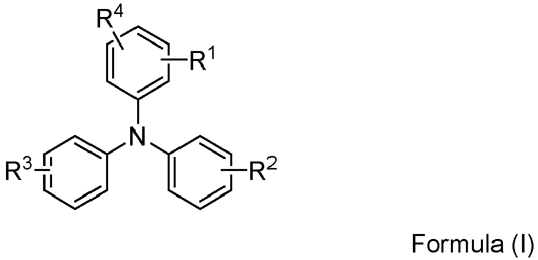 wherein:
- R¹ is chosen from the group comprising: 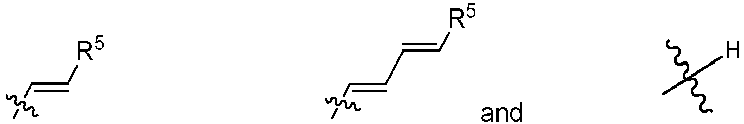
- R² is chosen from the group comprising: 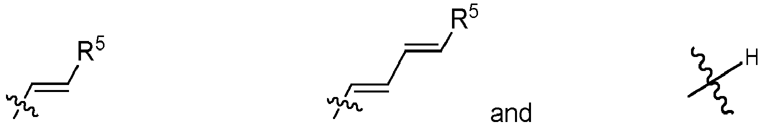
- R³ is chosen from the group comprising: 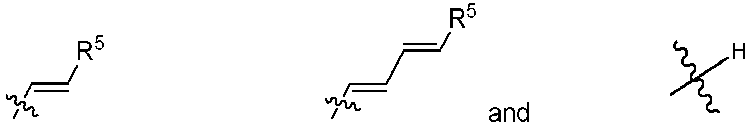 with R⁵ being chosen from the group comprising a heterocyclic moiety having 5 or 6 members and a heterocyclic compound having 5 or 6 members condensed with a benzene ring,
- R⁴ is chosen from the group comprising a hydrogen, an alkyl or a polyethylene glycol chain, terminated by a function chosen among OH, NH₂, COOH, OR and COOR.

The term "heterocyclic compound having 5 or 6 members" refers herein to cyclic compounds having as ring members atoms of at least two different elements. More particularly, the heterocyclic compounds are organic cyclic compounds, so they comprise at least one carbon molecule and at least one heteroatom chosen among nitrogen, oxygen and sulfur. The heterocyclic compound may be chosen among pyridine, oxazole, thiophene, furane, thiazole and imidazole and their derivatives.

The term "heterocyclic compound having 5 or 6 members condensed with a benzene ring" refers herein to heterocyclic compound having 5 or 6 members as defined above, derived by fusion with a benzene ring. Such condensed heterocyclic compounds may be chosen among benzothiazole, benzimidazole, quinoline, benzothiophene, benzoxazole and benzofurane. The benzene ring may comprise optionally a heteroatom chosen among nitrogen, oxygen and sulfur, as for example the naphthyridine moiety.

The term "alkyl" refers herein to univalent groups derived from alkanes by removal of a hydrogen atom from any carbon atom -CₙH₂ₙ₊₁. It refers especially to the groups derived by removal of a hydrogen atom from a terminal carbon atom of unbranched alkanes form a subclass of normal alkyl (n-alkyl) groups H(CH₂)ₙ. n may be any integer comprised from 1 to 12.

In cases where the alkyl chain is terminated by a function chosen among OH, NH₂ and COOH, the structure -CₙH₂ₙ₊₁ becomes -CₙH₂ₙ-OH, -CₙH₂ₙ- NH₂ and -CₙH₂ₙ- COOH respectively.

The term "polyethylene glycol chain" (also referred to as "PEG chain) refers herein to linear polyethers made from ethylene glycol monomers, having the structure H-(O-CH₂-CH₂)ₙ-OH, with n an integer comprised from 1 to 4. Advantageously, PEG chain may have a molar mass of less than 20,000 g/mol.

In cases where the PEG chain is terminated by a function chosen among NH₂ and COOH, the structure H-(O-CH₂-CH₂)ₙ-OH becomes H-(O-CH₂-CH₂)ₙ- NH₂ and H-(O-CH₂-CH₂)ₙ-COOH.

According to the invention, each of the substituents R₁, R₂ and R₃ may be in positions ortho, meta, or para on the benzene ring with respect to_the position of the nitrogen of the triphenylamine. Therefore, the compounds may be asymmetrical or symmetrical. When the compounds are symmetrical, the substituents R₁, R₂ and R₃ may all be in position ortho, or in position meta, or in position para. The pharmaceutical composition of the invention may also comprise, in some cases, a mixture of symmetrical and asymmetrical coumpounds.

In an embodiment, R¹, R² and R³ are identical and are chosen from the group comprising:

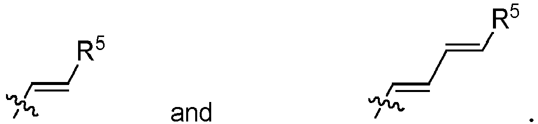

In another embodiment:
- R¹ and R² are identical and are chosen from the group comprising: 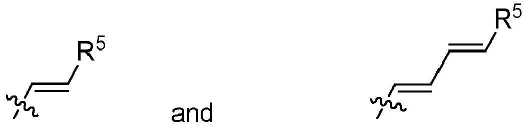
- R³ is

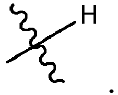

In another embodiment:
- R¹ is chosen from the group comprising: 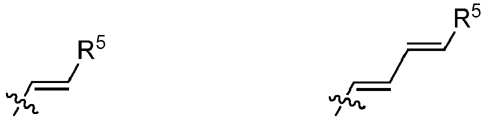
- R² and R³ are identical and are

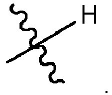

More particularly, the compound may be chosen among:

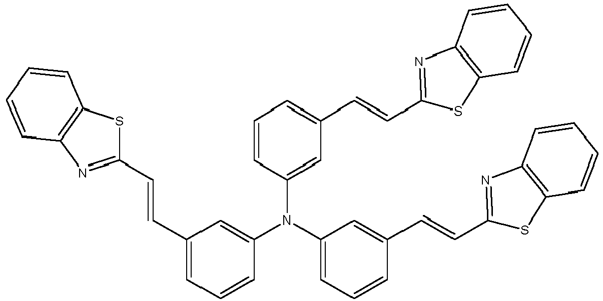

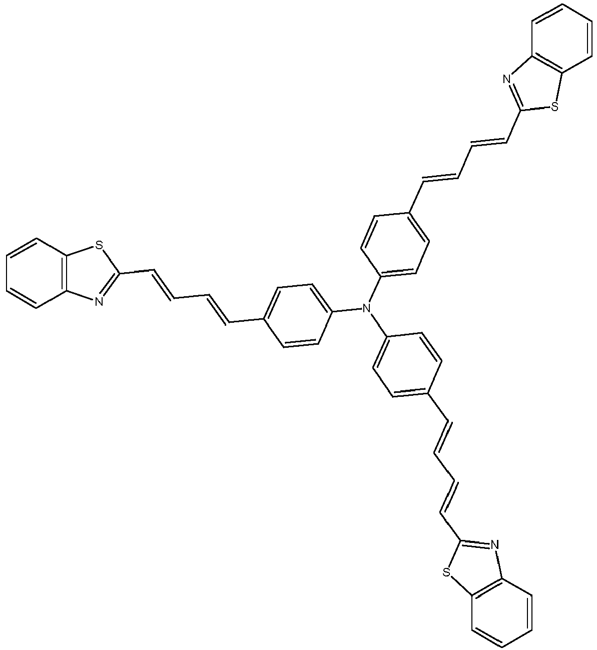

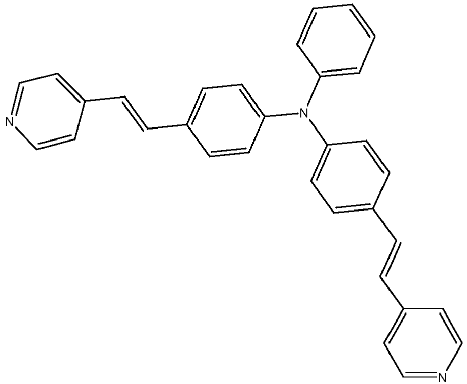

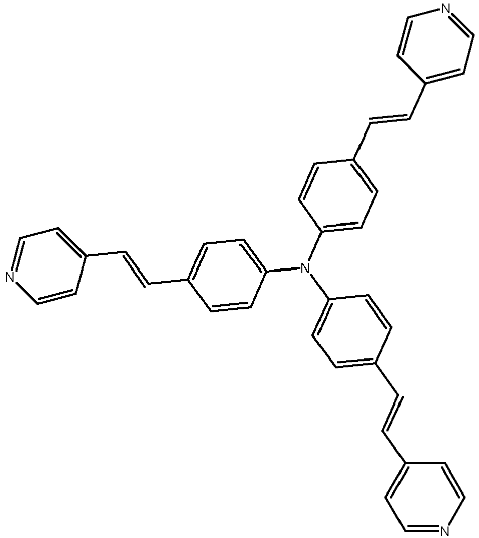

The compounds may be prepared by methods known in the art, for example in document WO2008/055969 ([1]), or in Guillaume Bordeau: « Nouvelles tryarylamines pour la microscopie biphotonique : application au marquage des acides nucléiques », Thesis, 2009 ([2]), or in Bordeau,G., Lartia,R. and Teulade-Fichou,M.-P. (2010) meta-Substituted triphenylamines as new dyes displaying exceptionally large Stokes shifts. Tetrahedron Lett., 51, 4429-4432 ([3]), or in Allain C. et al., 2007, Vinyl-pyridinium Triphenylamines : novel far-red emitters with high photostability and two-photon absorption properties for staining DNA ChemBioChem 8, 424-433 ([4]).

The term "pharmaceutical composition" refers herein to a composition comprising at least one active drug and at least one pharmaceutically acceptable excipient. According to the invention, one active drug is the compound as defined above. Advantageously, the pharmaceutical composition may comprise at least one other active drug, for example chosen among dexamethasone, remdesivir, azithromycin. The pharmaceutically acceptable excipient may be any substance adapted to be formulated alongside the active ingredient (s) of the pharmaceutical composition of the invention. It may be for example selected among antiadherents, binders, coatings, colours, disintegrants, flavors, glidants, lubricants, preservatives, sorbents, sweeteners and vehicles.

The pharmaceutical composition may be in any pharmaceutical form, i.e. enteral, parenteral or topical. It may be a medicament for oral administration, for example selected from the group comprising a solution, a syrup, a suspension, an emulsion and oral drops. Alternatively, it may be a medicament in the form of an oral effervescent dosage form, selected from the group comprising tablets, granules, powders. Alternatively, it may be an oral powder or a multiparticulate system, in a form selected from the group comprising beads, granules, mini tablets and micro granules. Alternatively, it may be a medicament in the form of an orodispersible dosage form, selected from the group comprising orodispersible tablets, lyophilised wafers, thin films, a chewable tablet, a tablet and a capsule, a medical chewing gum. Alternatively, it may be a medicament for buccal and sublungual routes, for example selected from the group comprising buccal or sublingual tablets, muco adhesive preparation, lozenges, oromucosal drops and sprays. Alternatively, it may be a medicament for topical-transdermal administration, for example selected from the group comprising ointments, cream, gel, lotion, patch and foam. Alternatively, it may be a medicament for nasal administration, for example selected from the group comprising nasal drops, nasal spray, nasal powder. Alternatively, it may be a medicament for rectal administration, for example suppository or hard gelatin capsule. Alternatively, it may be a medicament for parenteral administration, for example subcutaneous, intramuscular, intravenous administration.

The pharmaceutical composition of the invention may be prepared using conventional techniques known by the skilled person. For example, the bulk drug substance may be dissolved in a suitable solvent in the presence of one or more of the excipients described above, and mixed.

In a second aspect, the present invention relates to the pharmaceutical composition as defined above, for use as a medicament.

Advantageously, the medicament may be administered so as to allow delivery of a pharmaceutically acceptable and efficient dose for the treatment of an infection, especially of a viral infection. For example, the administration may comprise a dose of 1 to 20 mg/kg of body weight per day. The administration may be carried out with one dose or with a plurality of doses per day.

In a more specific aspect, the present invention relates to the pharmaceutical composition as defined above, for use in the prevention or the treatment of an infection. It may be for example an infection caused by a virus for which viral entry into cells requires the interaction between the spike S protein of the virus and the Angiotensin-Converting enzyme 2 ACE2 at the surface of the cell membrane. Advantageously, the pharmaceutical composition may be used for inhibiting the interaction between Spike and ACE2 proteins.

The virus may be, for example, selected from the Coronaviridae family. Viruses of the Coronaviridae family may be for example selected from the group comprising Coronavirus 229E, Coronavirus NL63, Coronavirus OC43, Coronavirus HKU1, Coronavirus SARS, Coronavirus MERS-CoV and Coronavirus SARS-CoV-2.

In a preferred embodiment of the invention, the viral infection is a SARS-CoV-2 infection. The SARS-CoV-2 may be the original strain appeared in 2019, or variants appeared from then, including alpha variant, beta variant, gamma variant and delta variant.

In another aspect, the present invention provides the compound as defined above, for use as a medicament, and in particular for use in the prevention or the treatment of an infection as defined in relation with the pharmaceutical composition. Especially, the compound may be for use in the treatment of SARS-CoV-2 infection as defined above.

This invention is further illustrated by the following examples with regard to the annexed drawings that should not be construed as limiting.

### Brief description of the figures

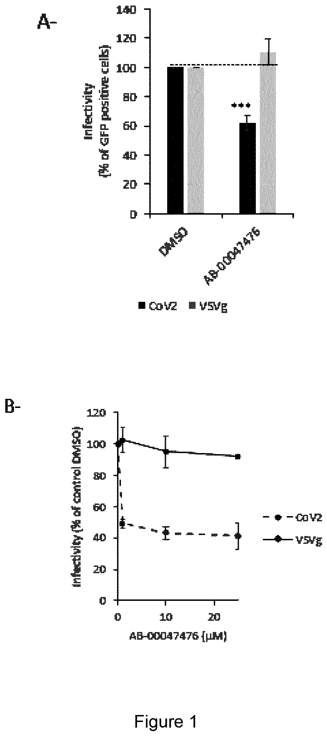

- Figure 1: represents TP m 3BTZ (named AB-00047476) compound was selected for further characterization using increasing concentration of drugs (B). Data are shown as means of at least three independent experiment ±standard deviation. ***p<0.001, **p<0.01.

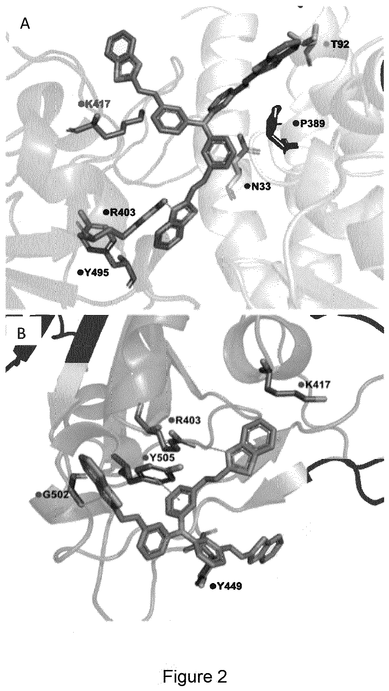

- Figure 2: represents the molecular docking of TP m 3BTZ compound to the spike/ACE2 complex and to the spike protein alone. Data were obtained from the predicted binding poses of TP m 3BTZ compound to the S-RDB and to S-RDB/ACE2 interface. The interacting amino acid residues have been highlighted. (A) TP m 3BTZ compound bound to the spike/ACE2 complex; (B) TP m 3BTZ compound bound to the spike alone.

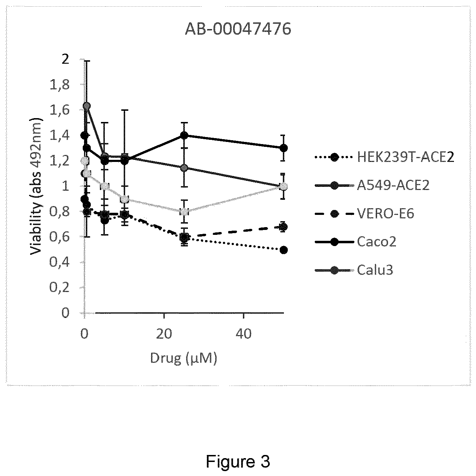

- Figure 3: represents cytotoxicity of TP m 3BTZ compound (named AB-00047476) in various cell lines (HEK293T-ACE2, A549-ACE2, VERO-E6, Caco2, Calu3). The viability of the cells (492nm absorbance in MTT assay) was plotted against the increasing concentration of drug. Data are shown as means of at three independent experiments. Data are shown as means of at least three independent experiments ± (SD).

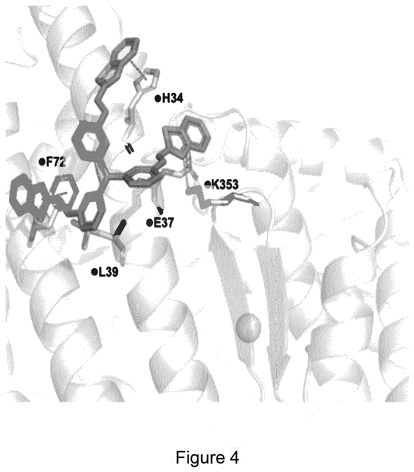

- Figure 4: represents molecular docking of TP m 3BTZ compound and the ACE2 alone. Data were obtained from the predicted binding poses of the compounds to the ACE2 around the interface region. The interacting amino acid residues have been highlighted. TP m 3BTZ compound bound to ACE2 alone.

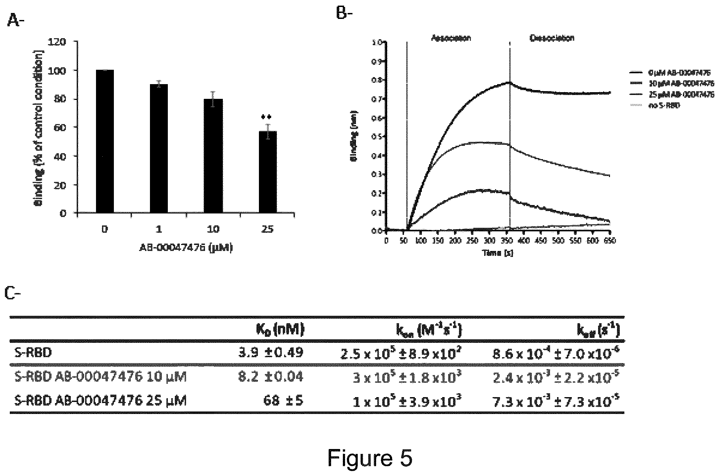

- Figure 5: represents the effect of TP m 3BTZ compound (named AB-00047476) on the *in vitro* S-RBD/ACE2 interaction using AlphaLISA and Bio-Layer Interferometry (BLI) technologies. SARS-CoV-2 S-RBD(His)₆ and human ACE2-Biot interaction have been first monitored by AlphaLISA (A) using 3 nM of each protein. Increasing concentrations of TP m 3BTZ compound were incubated for 30 min with S-RBD(His)₆ before being mixed with ACE2-Biot for 2h. The microplate was read after 2 h incubation with anti-6X His acceptor and streptavidin donor beads. Data obtained with the compounds were compared to DMSO control and are reported as mean of percentage of control condition from two to three independent experiments in duplicate ± standard deviation. **p<0.01. The effect of TP m 3BTZ compound on the binding kinetics of S-RBD to biotinylated ACE2 immobilized on streptavidin biosensor was also performed using BLI experiments (B). Baseline with reaction buffer was measured for 60 s. For the association step (300 s), each loaded biosensor was dipped into 50 nM of S-RBD(His)₆ preincubated for 15 min with increasing concentrations of TP m 3BTZ compound. The dissociation step was subsequently measured for 300 s. Sensorgrams curves shown in (B) were plotted using Prism 5.0 software (Graphpad Software, La Jolla, CA). The association and dissociation experimental curves were local fitted using a 1:1 binding model with the Blitz pro 1.1 software. Kinetic binding parameters (kₒₙ, k_{off}) and the equilibrium dissociation constant (K_{D}) were determined as means of two to three independent experiments and reported in (C). Data are shown as means of two to three independent experiment ±standard deviation. ***p<0.001, **p<0.01.

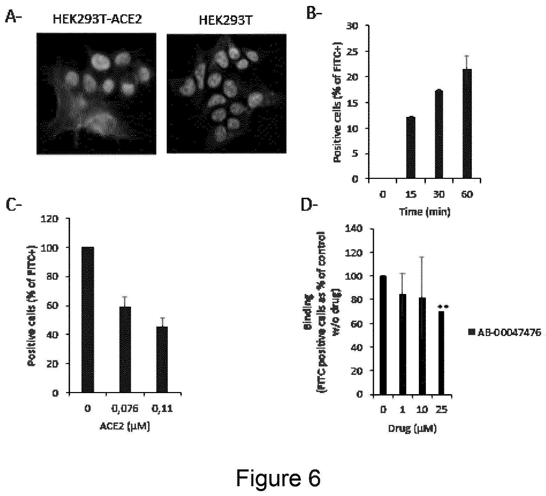

- Figure 6: represents the effect of TP m 3BTZ compound (named AB-00047476) on the S-RBD/ACE2 interaction in cellular context. The SARS-CoV-2 S-RBD fragment was added to either HEK293T or HEK293-ACE2 cells for 0-60 minutes and immunofluorescence staining was performed using anti-(His)₆ antibody and secondary antibody coupled to Alexa-Fluo 488. Cells were observed by epifluorescence microscopy (A). Cells were also analyzed using flow cytometry to detect the percentage of positive cells for FITC signal during time (B). Increasing concentrations of either soluble ACE2 (C) or TP m 3BTZ compound (D) were added with the RBD. Data are reported as mean of percentage of positive cells from at least three independent experiments. **p<0.05.

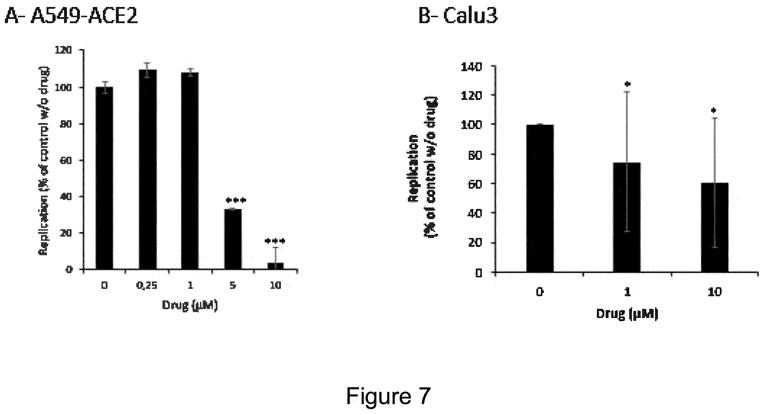

- Figure 7 represents: the effect of the TP m 3BTZ compound on SARS-CoV-2 replication in human pulmonary cells. A549-ACE2 (A), or Calu3 (B) cells were incubated with the SARS-CoV-2 reference strain (MOI=1) and increasing concentrations of drugs. The replication was evaluated by the quantification of the viral genome copies after 24 hours. Data are reported as means from 3-4 independent experiments and expressed as a percentage of control w/o drug.

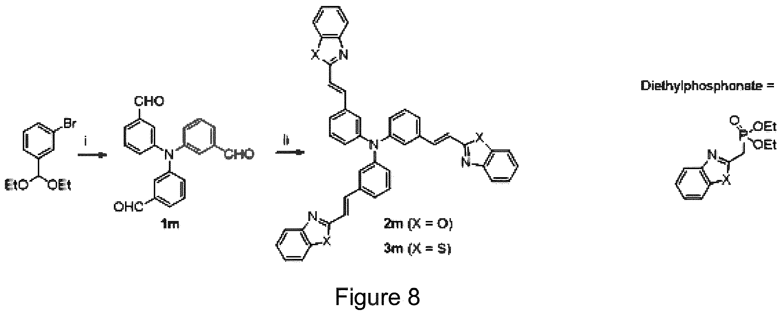

- Figure 8 represents the synthesis scheme of meta triphenylamine derivatives. Reagents and conditions are as described in example 4.

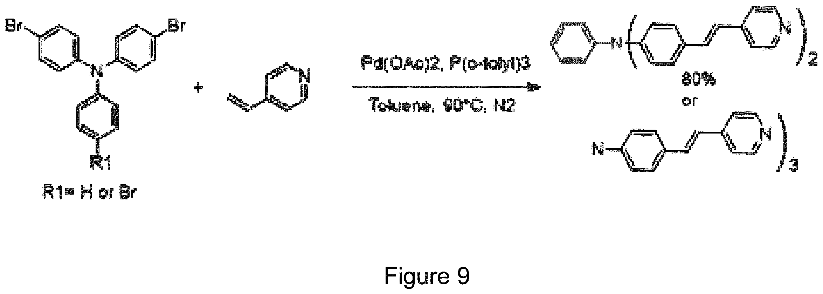

- Figure 9 represents the synthesis scheme of para pyridine derivatives as described in C. Allain et al. ([4]).

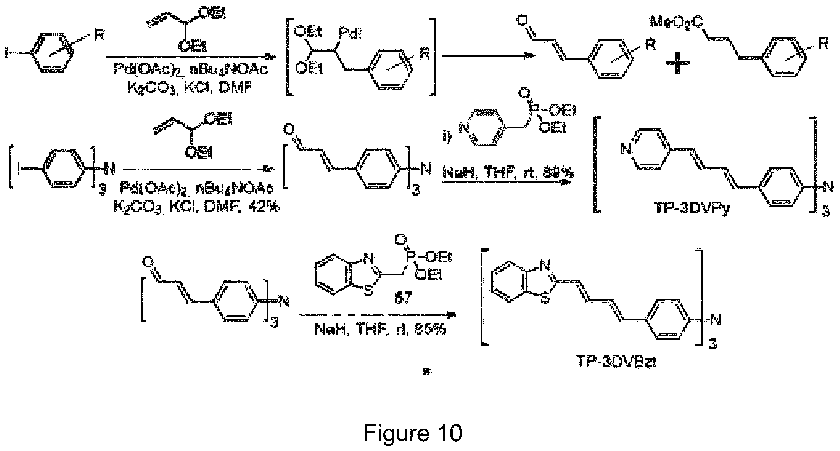

- Figure 10 represents the synthesis scheme of divinyl para derivatives as described in Guillaume Bordeau PhD Thesis ([2]).

### Examples

### Example 1: preparation of 4,4'-Bis[2-((E)-pyridin-4-yl)vinyl]-triphenylamine

The compound 4,4'-Bis[2-((E)-pyridin-4-yl)vinyl]-triphenylamine, which corresponds to the compound of general formula: 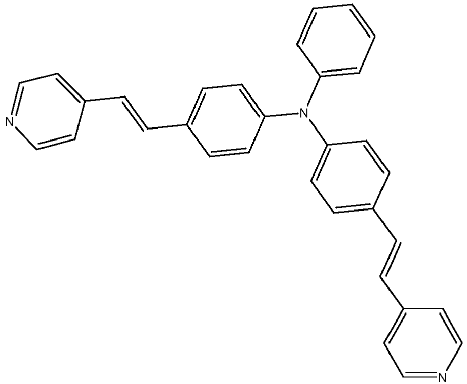 is synthesized as described in patent application WO2008/055969 [(1)].

In dry acetonitrile (6 mL), 4,4'-diiododiphenylamine (406 mg, 1 eq.) and 2-(trimethylsilyl)benzene trifluoromethanesulphonate (244 mL, 602 mg, 1.04 eq.) are dissolved. After stirring for 1 minute, finely ground CsF is added (295 mg, 2.01 eq.) and the resulting suspension is stirred for 3 days protected from light. Next, the solution is evaporated, taken up in n-hexane and filtered on silica. After evaporating the mother liquors, compound 2 (4,4'-diiodotriphenylamine) is isolated in the form of a fine white powder (yield: 86%).

Compound 2 (4,4'-diiodotriphenylamine) (108 mg, 220 mmol), palladium acetate (6 mg, 12%) and tris-(o-tolyl)phosphine (15 mg, 22%) are introduced into a dry reactor which is then purged with nitrogen. Then 70 µL of 4-vinylpyridine (660 mmol) and 3 mL of a degassed triethylamine/dimethylformamide mixture (TEA/DMF: 2/1, v/v) mixture are added successively. The mixture is stirred at 85-90 °C, under nitrogen for 3 hours. Next, its temperature is brought to room temperature and the solvents were evaporated under high vacuum. The oily residue was diluted with dichloromethane and washed with water several times. Then the organic phase is dried and concentrated. The oily residue is purified by chromatography on silica gel (elution: dichloromethane/methanol 100/0 to 95/5, v/v). 79 mg (200 mmol) of 4,4'-Bis[2-((E)-pyridin-4-yl)vinyl]-triphenylamine was obtained in the form of an orange powder (yield: 80%).

### Example 2: preparation of 4,4',4"-Tris[2-((E)-pyridin-4-yl)vinyl] triphenylamine

The compound 4,4',4"-Tris[2-((E)-pyridin-4-yl)vinyl] triphenylamine, which corresponds to the compound of general formula: 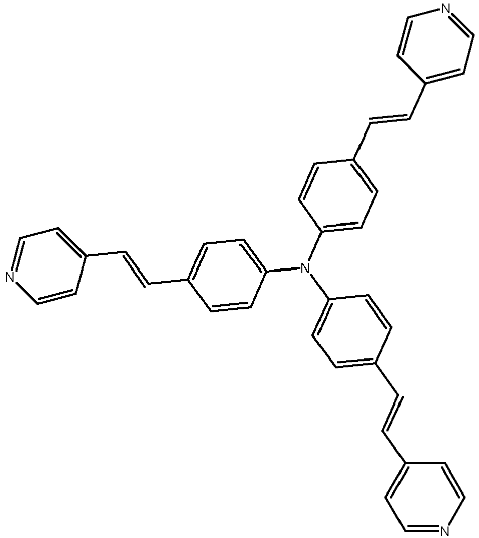 is synthesized as described in patent application WO2008/055969 ([1]).

Tris-(4-bromophenyl)amine (Sigma-Aldrich) (1 g , 2.07 mmol, 1 eq.), palladium acetate (23 mg, 0.104 mmol, 0.05 eq.) and tris-o-tolylphosphine (95 mg, 0.311 mmol, 0.15 eq.) are introduced into a dry reactor which is then purged with nitrogen. One mL of 4-vinylpyridine (0.98 g, 9.32 mmol, 4.5 eq.) and 5.4 mL of a TEA/DMF mixture (2/1, v/v) are added successively. The mixture is stirred at 85-90 °C, under nitrogen for 25 hours. Then its temperature is brought to room temperature and the solvents are removed under high vacuum. The product is diluted with 120 mL of dichloromethane, washed with 3 x 30 mL of a saturated sodium carbonate solution and dried. The volume of dichloromethane is reduced and the product is precipitated by adding hexane and cooling to 4°C. 0.943 g of 4,4',4"-Tris[2-((E)-pyridin-4-yl)vinyl] triphenylamine is thus obtained in the form of an orange powder (yield: 85.5%).

### Example 3: preparation of Tris{4-[(1E,3E)-4-(1,3-benzothiazol-2-yl)buta-1,3-dien-1-yl]phenyl}amine

The compound TP-DV-3BZT, which corresponds to the compound 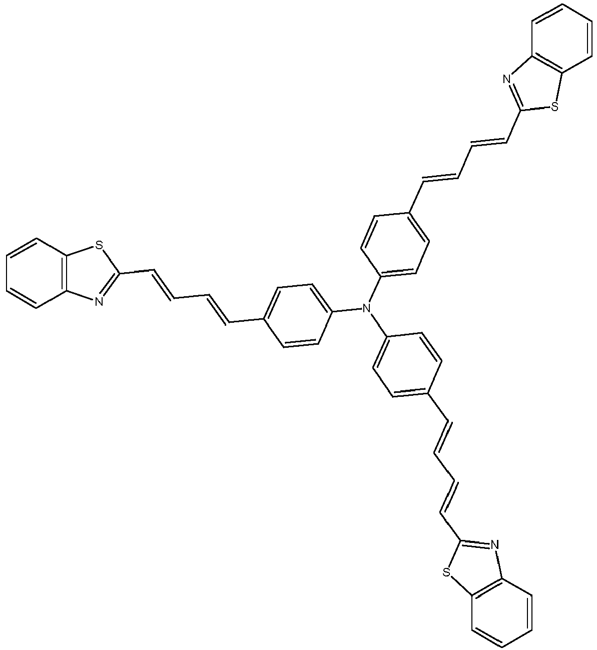 is synthesized as described in the thesis of Guillaume Bordeau: « Nouvelles triarylamines pour la microscopie biphotonique : application au marquage des acides nucléiques », Thesis, 2009 ([2]).

Acrolein diacetal (1.1 mL, 7.2 mmol, 9 equiv.), tetrabutylammonium acetate (1.2 g, 4 mmol, 5 equiv.), potassium carbonate (500 mg, 3.6 mmol, 4.5 equiv.), potassium chloride (180 mg, 2.4 mmol, 3 equiv.) and palladium diacetate (16 mg, 0.7 mmol, 0.09 equiv.) were successively added to a solution of trisiodotriphenylamine (500 mg, 0.8 mmol, 1equiv.) in degazed dry DMF (10 mL) under argon atmosphere. The reaction mixture was stirred for 8 h at 90°C, quenched with 10 mL of HCI 2N and stirred for 30 minutes, and then diluted with dichloromethane. After decantation, the organic layer was washed twice with water, dried over MgSO₄, filtered and concentrated to dryness. The crude residue was purified by column chromatography (gradient eluent Cyclohexane/ethyl acetate 80/20 to 70/30) to afford the desired Tris (4-formylethenylphenyl)amine in a pure form , 41% yield (136 mg, 2.6 mmol) as a yellow powder.

In dry THF, Diethyl-benzothiazolylphosphonate (126 mg, 0.44 mmol, 3.1 equiv.) and sodium hydride (20 mg, 0.49 mmol, 3.5 equiv.) were added. After 15 min stirring Tris(4-formylethenylphenyl)amine (60 mg, 0.15 mmol, 1 equiv) in dry THF (5 mL) was added to the solution and the resulting mixture was stirred at room temperature for 48h in the dark. Water and dichloromethane were added, after decantation the aqueous layer was extracted 3 times with dichloromethane and the organic layer was washed with brine, dried over MgSO₄, filtered and concentrated to dryness. The crude residue was purified by dissolution in a minimal amount of dichloromethane and precipitated with pentane and/or diethyl ether and filtered to afford the desired compound Tris{4-[(1E,3E)-4-(1,3-benzothiazol-2-yl)buta-1,3-dien-1-yl]phenyl}amine TP-DV-3BZT as a bright orange powder in 85% yield (100 mg, 0.12 mmol).

### Example 4: preparation of Tris {3-(2-(benzothiazole-2-yl)vinylphenyl}amine)

The compound TP m 3BTZ, which corresponds to the compound: 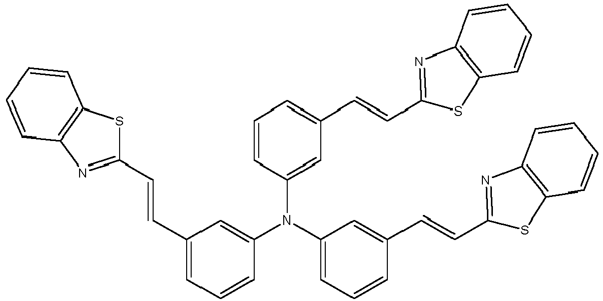 is synthesized as described in G. Bordeau et al. (2010) ([3]) and shown in figure 8.

Preparation of the trisadehyde precursor: Tris-(dibenzylideneacetone)dipalladium Pd₂₍dba)₃ (121 mg, 0,132 mmol,3%) and 2-(di-Tert-butylphosphino)biphenyl P(C₁₂H₉)(t-Bu)₂ (100mg, 0.335mmol, 7.5%) were suspended in dry and degassed dioxane (4ml). After stirring for 15 min at r.t., 3-bromobenzaldehyde diethylacetal (2.85 ml, 13.98 mmol, 3.2 equiv), lithium amide (100mg, 4.35 mmol, 1 equiv.) and sodium tert butoxide (1.25g, 13.06mmol, 3 equiv.) were added and the resulting suspension was heated at 85°C for 30h. Then Tris-(dibenzylideneacetone)dipalladium Pd₂₍dba)₃ (121 mg, 0,132 mmol,3%) and 2-(di-Tert-butylphosphino)biphenyl P(C₁₂H₉)(t-Bu)₂ (100mg, 0.335mmol, 7.5%) were added and the mixture heated for 16 additional hours. The mixture was cooled down to room temperature, diluted in dichloromethane and washed with hydrochloric acid (1M) and water. After decantation the organic layer was dried over MgSO₄, concentrated to give a yellow oil that was purified by column chromatography (n-hexane/CH₂Cl₂ 1:1-1:0, v/v). Trituration of the residue in ether Et₂0 followed by filtration afforded Tris (3-formyl phenyl)amine in 25% yield (363mg, 1.0mmol).

Preparation of compound TPm-3BZT : In dry THF (15ml) were added diethyl [(benzothiazole-2-yl) methyl] phosphonate (288mg, 1.0 mmol , 3.3 equiv.) and sodium hydride NaH (27mg, 1.12 mmol, 3.7 equiv, 60% mineral oil) . After 15 min stirring a solution of Tris (3-formyl phenyl)amine ( 100mg, 0.304 mmol, 1 equiv.) in THF ( 5mL) was added and stirred at room temperature for 48h in the dark. Water and dichloromethane were added and the organic layer washed with brine, dried over MgSO₄ and concentrated to dryness. The crude residue was purified by dissolution in a minimal amount of dichloromethane followed by precipitated with pentane and filtered to afford compound Tris {3-(2-(benzothiazole-2-yl)vinylphenyl}amine)-TPm-3BZT as yellow powder 64 % yield ( 140mg, 0.195 mmol).

### Example 5: effects of triphenylamines as new antiviral drugs targeting multiple SARS-CoV S/ACE2 interfaces

### In cellulo screen for specific S/ACE2-mediated SARS-CoV-2 entry pathway.

TP m 3BTZ compound of formula: 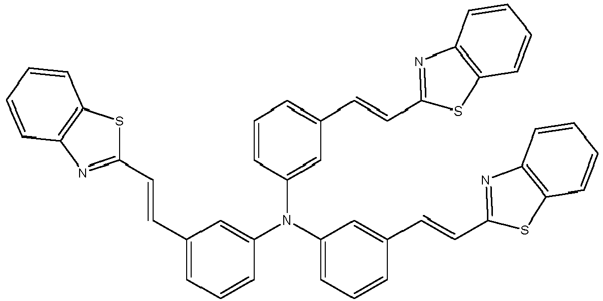 were first tested on the lentiviral vectors-based cellular approach previously set up in the laboratory (Lapaillerie et al. (2021) ([5])). Briefly, lentiviral vectors pseudotyped with the SARS-CoV-2 Spike protein (CoV-2 LV) have been produced from HEK293T cells transfected with three plasmids: a plasmid encoding a lentiviral backbone (LV44: pRRLSIN-PPT-hPGK-eGFP-WPRE) expressing a marker protein, the HDM_IDTSpike_fixK plasmid expressing the Spike protein (kind gift from the Bloom laboratory (Crawford et al. (2020) ([7])) and the psPAX2 plasmid expressing the other HIV proteins needed for virion formation (Tat, Gag-Pol, and Rev). HEK293T-ACE2 cells containing 9 copies of the *ACE2* gene were generated by lentiviral vector insertion of the human *ACE2* gene in their genome ([5]). The CoV-2 LVs were shown to transduce efficiently only the 293T-ACE2 cells in contrast to the original 293T cells leading to the integration and expression of the *eGFP* gene encoded by the LV (data not shown). This confirms that the transduction efficiency monitored by the percentage of eGFP positive cells relies on the early S/ACE2 interaction. On the contrary, typical VSVg pseudotyped LV were able to transduce both 293T and 293T-ACE2 cells and will serve as control for the next selection of drugs specific for S/ACE2 entry. Inhibition competition experiments using the soluble ACE2 protein fully validated the S/ACE2 dependency of the LV CoV-2 infectivity while the LV VSVg was not affected by the protein (data not shown) as previously shown ([5]).

TP m 3BTZ compound was thus tested on both VSVg and SARS-CoV-2 LVs infectivity to determine its possible effect on the S/ACE2 mediated entry pathway. A first screen using 10 µM concentration of drug showed significant inhibitory effect for some molecules on CoV-2 LVs without any effect on VSVg LVs (see Figure 1A). TP m 3BTZ compound was found the most efficient by inducing a 40 to 80% decrease of the viral infectivity. Further accurate analysis of the inhibitory effect of the compound using increasing concentrations confirmed its specific capability of inhibiting the CoV-2 LVs infectivity (Figure 2B) without significant effect on the VSVg LVs within the 0-20µM range. The cytotoxicity of the two molecules determined in different cell lines including HEK-293T, A549, Calu3 and Caco2 cell lines using MTT assay showed no or poor cytoxoxicity within the 0-50µM range (Figure 3).

Taken together our data strongly suggested that TP m 3BTZ compound interfered with the early S/ACE2 mediated SARS-CoV-2 entry process. To confirm this, we next investigated whether this inhibitory effect could be due to direct effect of the drug on the physical interaction between S and ACE2 using molecular docking and biochemical approaches.

### Molecular docking analysis of the drugs interactions with S and ACE2

To better understand the binding of TP m 3BTZ compound to the target protein, molecular docking studies were performed. Figure 2 displays results from the predicted binding poses to the S-RDB/ACE2 interface and to the S-RBD, highlighting the interacting amino acid residues.

The docking of TP m 3BTZ compound to the S-RDB/ACE2 interface (Figure 2B) resulted in a score of 100.71 (GOLD/PLP). The most favored pose suggests that this molecule forms one hydrogen bond through the nitrogen of one of its thiazole groups and S-RDB Arg403, with a distance of 2.14 Å. In addition, TP m 3BTZ compound also establishes hydrophobic interactions with ACE2 Asn33, Thr92 and Pro389 and with S-RDB Lys417 and Tyr495.

The docking of TP m 3BTZ compound to the S-RDB protein alone (Figure 2D) resulted in a score of 89.58. The most favored pose of this molecule reveals two hydrogen bonds. One between Arg403 and the nitrogen from one of the thiazole groups, and another between Gly502 and the nitrogen from another of the thiazole groups, with lengths of 2.28 Å and 2.26 Å, respectively. There is also the formation of π-π stacking interactions between Tyr505 and one of the phenyl groups in the centre of the molecule. Residues Arg403, Lys417, Tyr449 and Tyr505 from S-RDB are also involved in hydrophobic interactions with the molecule. The interactions of TP m 3BTZ compound with Tyr449, Gly502 and Tyr505 from S-RDB are of special relevance as these residues have been shown to be important for the formation of the S-RDB/ACE2 complex. A previous MD simulation (5) of the S-RDB/ACE2 complex has shown that these residues of the spike are involved in important hydrogen bonds with ACE2. Tyr449 interacts with ACE2 Asp38, Gly502 interacts with ACE2 Lys353 and Tyr505 interacts with ACE2 Glu37. All of these are major interactions, being present during 54 %, 57 % and 56 % of simulation time, respectively. The results suggest that TP m 3BTZ compound binding to S-RDB would prevent the normal S-RDB/ACE2 association.

Additionally, the ability of TP m 3BTZ compound in binding to ACE2 competitively with the spike protein was evaluated. Both molecules were docked against the ACE2 receptor, defining as potential binding region the surface of the ACE2 that is known to interact and recognize the spike protein. Results are presented in Figure 4, showing that TP m 3BTZ compound can bind ACE2 with strong binding scores that are comparable to those observed when docked to S-RDB/ACE2 interface and to S-RDB alone.

The docking of TP m 3BTZ compound to ACE2 alone yielded a score of 78.19. The most stable pose suggests the formation of one hydrogen bond with a length of 2.39 Å between Lys353 and the nitrogen from one of the thiazole groups. TP m 3BTZ compound and ACE2 also interact via π-π stacking interactions. These interactions are observed between ACE2's His34 and another of the thiazole groups, and between Phe72 and the third thiazole group, with lengths of 3.47 and 4.57 Å respectively. Hydrophobic interactions between the TP m 3BTZ compound and ACE2's Glu37, Leu39 and Phe72 are also well defined. As has been the case for most the docking results here shown, there are some interactions featuring residues that have been reported to form hydrogen bonds between ACE2 and S-RDB. In this case, the interactions between TP m 3BTZ compound and ACE2 Glu37 and Lys353 are the particularly noteworthy interactions. During the MD simulations of the S-RDB/ACE interface ([5]) Glu37 has been shown to form a hydrogen bond with S-RDB Tyr505 for 56 % of simulation time, while Lys353 forms a hydrogen bond with Gly502 for 57% of simulation time. The disruption of these interactions on the side of the ACE2 could further help explaining the inhibitory capabilities of this molecule.

### Effect on the S-RBD/ACE2 association

To confirm that TP m 3BTZ compound may physically prevent the formation of the S/ACE2 complex we next performed an *in vitro* alphaLISA assay using the recombinant S minimal binding domain (RBD) fused to a 6x-His tag (S-RBD(His)₆) and human ACE2 protein (ACE2-Biot).

For this, the AlphaLISA assay previously set up ([5]) was used and the binding conditions were adapted for use in 384-well microplate in a final reaction volume of 20 µl. The optimal concentration of 3 nM for each recombinant protein was previously established with cross-titration experiments. For the competition assay, increasing concentrations (1 to 25µM) of TP m 3BTZ compound were preincubated with S-RBD(His)₆ in the plate before being mixed with ACE2-Biot for 2h. Anti-6XHis acceptor and streptavidin donor beads were used for complex capture after 2 h of incubation of the two partners. As reported in Figure 5A, the AlphaLISA data confirmed that TP m 3BTZ compound could interfere with the formation of the RBD/ACE2 complex reaching in both cases 50% inhibition at 25 µM of drugs. TruHIts counterassay experiment did not show any significant effect of the compound on the AS signal confirming the specific inhibition of the S-RBD/ACE2 interaction (data not shown). In order to get additional information about the effect of the drugs on the kinetic of the S-RBD/ACE2 interaction we performed biolayer interferometry (BLI) experiments as setup previously ([5]). In these competition binding assays, biotinylated ACE2 was loaded onto streptavidin biosensors and binding kinetics were carried out with 50 nM of S-RBD(His)₆ preincubated with increased concentrations of TP m 3BTZ compound as described in the Materials and Methods section. A strong binding of S-RBD(His)₆ to ACE2 was observed as indicated by the wavelength shift in the BLI sensorgrams (Figure 4B). The kinetic binding parameters (kₐ, k_{d}) and equilibrium dissociation constant (K_{D}) were determined after local fitting of the association and dissociation curves using a 1:1 binding model with ForteBio Blitz pro 1.1 software. The K_{D} was found to be 3.42 ±0.49 nM confirming previous data (([5]), ([6])). Addition of TP m 3BTZ compound inhibited the interaction leading to a strong drop of the K_{D} to 111 ±16 nM and 68.8 ± 5.1 nM respectively (see Figure 5D). Determination of the kinetic constants in the absence or in the presence of drugs showed that the compounds interfered on both the S-RBD/ACE2 association and dissociation steps, indicating that they can not only prevent the formation of the S-RBD/ACE2 complex and also decrease its stability, in agreement with the docking models.

In a cellular context both ACE2 and S are embedded in a membranous environment, i.e. the viral envelop and the cellular membrane. Thus, we next investigate whether the compounds could also prevent the S/ACE2 interaction in a more physiological context. For this purpose we used the cellular S-RBD/ACE2 interaction model developed previously ([5]). This model allows to monitor the association between recombinant S-RBD protein and the cellular membranous ACE2 protein at the HEK293T-ACE2 cells surface by anti-S immunofluorescence (Figure 6A). Flow cytometry analysis allows the quantification of the global S-RBD association to ACE2 protein (Figure 6B) and competition with soluble ACE2 protein confirms the specificity of the interaction as well as validates the assay for testing potential S/ACE2 inhibitors (Figure 5C). Addition of TP m 3BTZ compound decreased the efficiency of S-RBD/ACE2 association at the surface of the cell (Figure 6D). The efficiency of TP m 3BTZ compound in infectivity assays is reported in Figure 1.

Altogether, the data obtained demonstrate that the two selected drugs block the early interaction between S and ACE2 supporting, in concert with the data obtained with pseudoviruses, their effect on the early entry pathway mediated by this interaction.

### Effect of TP m 3BTZ compound on wild type SARS-CoV-2 infectivity on naturally susceptible human cells.

We next investigate whether this S/ACE2 inhibitory effect could be sufficient for blocking the replication of wild type circulating SARS-CoV-2 strains in different cell lines naturally susceptible to the infection.

TP m 3BTZ compound were tested in different pulmonary cell lines overexpressing, or not, the human ACE2 viral receptor and infected with the SARS-CoV-2 reference strain. As reported in Figure 6A, TP m 3BTZ compound induced an inhibition of the viral replication in A549-ACE2 cells with an EC₅₀ for TP m 3BTZ compound. TP m 3BTZ compound was then further tested in infection assays using the Calu3DFD human lung cancer cell lines that naturally express the ACE2 receptor. As reported in Figure 6B an inhibitory effect of the viral replication was confirmed for TP m 3BTZ compound leading to a significant 50% inhibition of the viral replication at concentration around 1µM.

As reported in Table 1, based on the apparent IC₅₀ and CC₅₀ reported in the different cell lines the TP m 3BTZ compound reached selectivity index (SI) higher than 50 making it good candidate and anti-SARS-coV-2 selective drug. We, thus, further studied the molecular mechanism underlying the viral inhibition by the TP m 3BTZ compound.

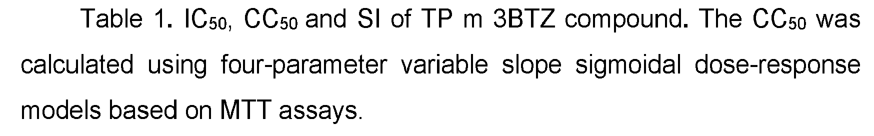

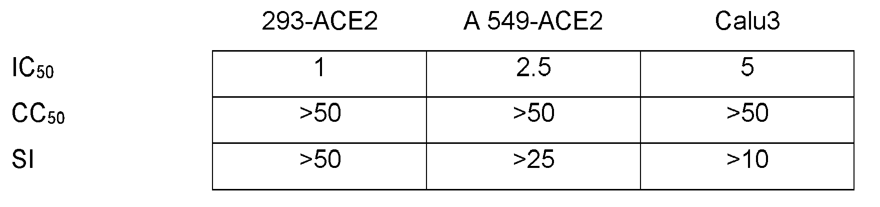

### Effect of TP m 3BTZ compound on SARS-CoV-2 circulating variants forms.

To determine whether TP m 3BTZ compound could be effective on the current major SARS-CoV-2 variants we first performed an *in silico* modelling and molecular dynamics simulations of the SARS-CoV-2 S/ACE2 complex variants alpha, beta, gamma and delta

Models for the S-RDB/ACE2 complex formed with the different SARS-CoV-2 mutants were prepared starting from the X-ray structure 6M0J (resolution 2.45 Å) (Lan et al. (2020) ([6])), which represents the original SARS-CoV-2 spike protein receptor binding domain complexed with ACE2. Mutant variants were prepared by modelling the interface mutations using the mutagenesis feature in Pymol 1.7.2.1 software, using the Dunbrack rotamer libraries available (Shapovalov and Dunbrack (2011) ([8])). Selection of the initial amino-acid conformation of each mutant was based on the dominant conformer predicted, excluding clashes with other residues. This computational mutagenesis protocol has been previously used with success in the study of other enzymes (Serrano et al. (2021) ([9]); Ferreira (2017) ([10])). The mutations modelled are described in Table 2.

**Table 2. Description of the SARS-CoV-2 mutants modelled in the evaluation of the S-RDB/ACE2 interaction**

| Mutant | Common Name | Pango Name | Mutations in the S-RDB |
|---|---|---|---|
| Alpha | UK | B.1.1.7 | N501Y |
| Beta | South Africa | B.1.351 | K417N, E484K, N501Y |
| Gamma | Brazil | B.1.1.28.1 | K417T, E484K, N501Y |
| Delta | India | B.1.617.2 | L452R, T478K |

All the 4 systems were subject to the same molecular mechanics minimization and molecular dynamics procedure previously described, employing AMBER and the ff14sb force field. Molecular dynamics simulations were run for 400 ns for each mutant. Final trajectories were analyzed in terms of backbone root-mean-square deviation (RMSD), hydrogen bonds formed and cluster analysis. From the cluster analysis performed on each mutant, a representative structure from the dominant cluster of conformations obtained for each mutant was selected using cpptraj from AmberTools and prepared for the molecular docking stage.

TP m 3BTZ compound was docked into the structures of each of the mutants using the PLP scoring function from GOLD with 500 independent genetic algorithm runs. Molecule was docked into: (1) the S-RDB/ACE2 interface; (2) S-RDB alone. Table 3 presents the best scores obtained when docking the TP m 3BTZ compound to the different variants, compared with the values obtained against the initial non-mutated S-RDB/ACE2.

**Table 3. GOLD/PLP docking scores of TP m 3BTZ compound to the structures of the different SARS-CoV-2 mutants**

| | TP m 3BTZ compound | |
|---|---|---|
| Targets | S-RDB/ACE2 | S-RDB |
| Initial | 100.71 | 89.58 |
| Alpha | 102.11 | 85.04 |
| Beta | 79.48 | 89.02 |
| Gamma | 90.71 | 92.70 |
| Delta | 93.84 | 93.01 |
| Average | 93.4 ± 8.1 | 89.9 ± 2.9 |

The results presented in Table 3 show that TP m 3BTZ compound is able to bind with high docking scores and consistently to the different mutants, reinforcing the idea that these molecules can be equally effective in impairing the S/ACE2 association in the different mutants.

### Analysis of the Interactions of TP m 3BTZ compound with S-RDB/ACE2, S-RDB alone and comparative docking to ACE2 alone.

One of the hypotheses emerging from the experimental studies was the ability of the selected molecules in binding simultaneously, targeting different components of the interface, namely (1) the S-RDB/ACE2 interface; (2) S-RDB alone; (3) ACE2 alone. It has been suggested that the combined effect of these three association modes could contribute for the inhibitory effect observed. To evaluate that hypothesis TP m 3BTZ compound, were docked into: (1) the S-RDB/ACE2 interface; (2) S-RDB alone; (3) ACE2 alone. For (2) and (3) the cavity for docking was defined based on the known binding region of the other partner in the complex formed (i.e. of ACE2 for the S-RDB model (2) and S-RDB for the ACE2 model (3), plus a radius of 10 Å. The PLP scoring function from GOLD was used with 500 independent genetic algorithm runs. Results are presented in Table 4.

**Table 4. GOLD/PLP docking scores of TP m 3BTZ compound to the different possible targets involved in the S-RDB/ACE2 association.**

| **Molecules** | **S-RDB/ACE2** | **S-RDB** | **ACE2** |
|---|---|---|---|
| TP m 3BTZ compound | 110.71 | 89.58 | 78.19 |

The results presented in Table 4 show that although the best docking scores are observed against the S-RDB/ACE2 interface, TP m 3BTZ compound exhibit high affinity towards S-RDB alone and interestingly towards ACE2 alone. Globally, these results agree with the proposed hypothesis that these molecules can target the different components involved in the S-RDB/ACE2 association, suggesting that the observed effect can results from the combined action of different binding modes to the interface and to the individual components.

Taken together all these data indicate that TP m 3BTZ compound is efficient on all the current SARS-CoV-2 strains and show an original entry inhibition mechanism by possibly acting simultaneously on distinct S/ACE2 interfaces.

### Discussion

The SARS-CoV-2 S/ACE2 mediated entry remains a major antiviral target not yet exploited in therapy. We identified TP m 3BTZ compound showing potent entry inhibition in lentiviral-based pseudovirus infectivity assays. The specific inhibition of SARS-CoV-2 LV infectivity in comparison with VSVg LVs suggested that TP m 3BTZ compound target the early S/ACE2 dependent entry process recapitulated in this model. The simplest explanation for the inhibition mechanism is a block in the S/ACE2 association due to binding of the drug on the S/ACE2 interface. Biochemical approaches confirmed a direct effect of TP m 3BTZ compound in blocking the S-RBD/ACE2 interaction. TP m 3BTZ compound was also shown to inhibit the interaction between the soluble S-RBD and the ACE2 receptor expressed at the surface of HEK-293T-ACE2 cells confirming its capability to interfere with the formation of the S/ACE2 complex in a physiological condition. Detailed analysis of the effect of the drugs on the kinetic parameters of the S/ACE2 complex formation indicated that it could both prevent the association between the two partners but also decrease the stability of the complex. These data are in full agreement with the molecular docking calculation performed on the S/ACE2 complex structure and on the S-RDB and ACE2 proteins alone indicating that both drugs could make contact with the viral and cellular proteins.

In order to determine whether TP m 3BTZ compound could be potential antiviral lead compound we tested it on the wild type viral replication using different cell lines including naturally susceptible cells. Data confirm that TP m 3BTZ compound was able to block or reduce the replication of the virus in pulmonary A549-ACE2 and Calu3 cells. Overall, TP m 3BTZ compound showed IC₅₀ between 0.25 to 5µM among all the cell lines without showing significant cyto-toxicity allowing them to reach selectivity indexes >10.

Interestingly, the molecular modelization studies reported in this work suggest that TP m 3BTZ compound may interact both with S and ACE2 protein. This fully agree with the BLI data indicating that the molecules could interfere the formation of the S/ACE2 complex as well as decreasing the stability of the formed complex. This may constitute an original inhibition mechanism of simultaneous blocking of various S/ACE2 interfaces increasing the inhibition potency in addition to decreasing the resistance emergency.

Apart, from the recently licensed remdesivir no effective drugs has been validated as potential COVID treatment. The compound reported here and its analogs constitute the first molecules acting on the SARS-CoV-2 entry step by targeting specifically the S/ACE2 interfaces making them very promising agent.

### MATERIALS and METHODS

### Molecular Docking of the LEAD compounds

For the molecular docking of TP m 3BTZ compound, the PLP scoring function from GOLD was used with 500 independent genetic algorithm runs. Molecules were docked into: (1) the S-RDB/ACE2 interface; (2) S-RDB alone; (3) ACE-2 alone. For (2) and (3) the cavity for docking was defined based on the known binding region of the other partner in the complex formed (i.e. of ACE2 for the S-RDB model (2) and S-RDB for the ACE2 model (3)), plus a radius of 10 Å. Analysis of the interactions was done with the help of Protein-Ligand Interaction Profiler (Adasme et al. (2021) ([11])).

### Chemicals

Triphenylamine synthesis procedures are described in ([3]) and ([4]).

The tris-benzothiazole triphenylamine derivative (purity >95% by Proton (1H) NMR spectrometer) and its chemical synthesis was previously described in (Bordeau,G. and Teulade-Fichou,M.-P. (2010) meta-Substituted triphenylamines as new dyes displaying exceptionally large Stokes shifts. Tetrahedron Lett., 51, 4429-4432 ([15])).

### Proteins and antibodies

SARS-CoV-2 (438-516) S-RBD(HiS)₆ including the minimal receptor binding motif previously described (([6]); Wrapp et al. (2020) ([12])) and biotynilated human ACE2 have been purchased from Fisher Scientific (respective references 16534204 and 16545164). Monoclonal anti-6X His tag antibody has been purchased from ABCAM (reference ab18184, dilution 1/200). Anti α-tubulin antibody has been purchased from SIGMA (reference T6199, dilution 1/500). Secondary goat anti-mouse antibody coupled to AlexaFluor 488 has been purchase from Fisher Scientific (Reference Allo2g, dilution 1/400).

### AlphaLISA binding assays

The AlphaLISA assay development was performed as previously described ([5]) using the recombinant S minimal binding domain (RBD) fused to a 6x-His tag (S-RBD(His)₆) and biotinylated human ACE2 protein (ACE2-Biot). The binding conditions were adapted for use in 384-well plate (white OptiPlate, reference 6007290, PerkinElmer, Waltham, MA) in a final reaction volume of 20 µl. An optimal final concentration of 3nM for each recombinant protein was previously established with cross-titration experiments. Proteins were diluted in the binding buffer (phosphate-buffered saline (PBS) pH 7.4, 0.1% (w/v) bovine serum albumin). For the AlphaLISA competition assay, increasing concentrations of TP m 3BTZ compound (1 to 25 µM, 1% DMSO final concentration) were preincubated with S-RBD(His)₆ for 30 min before being mixed with ACE2-Biot for 2h with rotation at room temperature. Next, 5µL of anti-6X His acceptor beads (Perkin Elmer, reference AL178) were added and after 1h of incubation at RT with rotation, 5 µL of streptavidin donor acceptor beads (Perkin Elmer, reference 6760002) were also mixed to the wells. This established a final concentration of 20 µg/mL for each bead. The plate was then incubated for 1h at RT in the dark before the AlphaLISA signal was detected using an EnSpire Multimode Plate Reader (Perkin Elmer). Negative control with binding buffer was used to control the assay quality. Data were analyzed with GraphPad Prism 5.01 software. The AlphaLISA binding data obtained with compounds were compared to the 1% DMSO control condition.

For hits validation, TruHits counterassay kit (reference AL900D, PerkinElmer) was performed by incubating TP m 3BTZ compound with biotin-BSA acceptor and streptavidin donor beads with the same working dilutions than in the AlphaLISA binding assay. In this validation assay, if the compound causes a decrease of the signal, it means that it interferes with AlphaLISA readout and therefore it is not relevant to the specific target of interest.

### Bio-Layer Interferometry binding assays.

Bio-Layer Interferometry (BLI) experiments were performed on a BLItz instrument (Sartorius, Göttingen, Germany) to measure the binding of S-RBD(His)₆ to ACE2-Biot. BLI assay was set up as previously described ([5]) with some adaptations for the competition binding analysis with drugs. All sample dilutions and baseline steps were carried out using the same reaction buffer (phosphate-buffered saline pH 7.4, 0.1% (w/v) bovine serum albumin, 1% (v/v) DMSO). Streptavidin biosensors (Sartorius, reference 18-5019) were first pre-wet for 10 min with the reaction buffer and 1 µM of ACE2-Biot was then loaded onto the coated biosensors for 500 s in order to reach a binding value of ∼ 3 nm. For the competition kinetic analyses, protein samples were prepared at the concentration of 50 nM S-RBD(His)₆ in accordance with our previous BLI binding studies and were preincubated for 15 min at room temperature with increased concentrations (0-25µM) of TP m 3BTZ compound. Binding kinetics were divided in three steps. Firstly, baseline with the reaction buffer was measured for 60 s. For the association step, each loaded biosensor was dipped into into S-RBD sample with drugs or DMSO as control for 300 s with a 2200 rpm shaking speed. Finally, the dissociation step of the bound S-RBD(His)₆ was monitored by dipping the biosensor back into the reaction buffer for 300 s. Systematic baseline drift correction was done by subtracting the shift recorded for biosensor loaded with ACE2 but incubated with reaction buffer. The association and dissociation experimental curves were local fitted using a 1:1 binding model with the Blitz pro 1.1 software and the kinetic binding parameters (kₒₙ, k_{off}) were determined as means of two to three independent experiments. The equilibrium dissociation constant (K_{D}) calculated as K_{D} = k_{off}/kₒₙ. Sensorgrams curves were plotted using Prism 5.0 software (Graphpad Software, La Jolla, CA).

### Cells and lentiviral vectors production

Lentivirus vector production was done by the service platform Vect'UB, (INSERM US 005 - CNRS UMS 3427- TBM-Core, Université de Bordeaux, France). Lentiviral particles were produced by transient transfection of HEK293T (human embryonic kidney cells according to standard protocols. In brief, subconfluent HEK293T cells were cotransfected with lentiviral genome (psPAX2) (gift from Didier Trono (Addgene plasmid # 12260 ), with an envelope coding plasmid (pMD2G-VSVG or wild type SARS-CoV-2 Spike protein (HDM_IDTSpike_fixK (kind gift from Bloom's laboratory, ([7]) and with vector constructs (44 :pRRLSIN-PPT-hPGK-eGFP-WPRE or pHAGE_EF1aInt_ACE2_WT) by calcium phosphate precipitation. LVs were harvested 48h posttransfection and concentrated by ultracentrifugation. Viral titers of VSV-g pseudotype pLV lentivectors were determined by transducing HEK293T cells with serial dilutions of viral supernatant and eGFP expression was quantified 5 days later by flow cytometry analysis. For SARS-CoV-2 Spike pseudotype, p24 antigen levels were measured in the concentrated viral supernatants by an enzyme-linked immunosorbent assay (Innotest HIV Ag nAb; Fugibio, France) and viral titers were estimated by comparing p24 antigen levels of each lentiviral supernatant with a similar VSV-g pseudotype lentiviral supernatant produced simultaneously.

HEK293T-ACE2 cell lines have been generated by lentiviral transduction using pHAGE_EF1aInt_ACE2_WT plasmid (kind gift from Bloom's laboratory ([7]). HEK293T cells (200 000 cells) were then transduced with optimized concentration of ACE2 lentiviral particles in 6-well plates. The efficacy of transduction was assessed using real time PCR ten days post infection. ACE2 provirus DNA from this cell line was quantified by q-PCR using the ΔCt method as compared with 1 copy cell line. DNA from two different cell clones (human 293T and K562 cells), containing a single integrated copy of the provirus, was used as a normalized cell line. HEK293T were cultured in DMEM medium supplemented with 1% penicillin-streptomycin and FBS (Fetal Bovine Serum) 10%.

A549 cells (Human alveolar basal epithelial carcinoma), Calu3 (human bronchial epithelial carcinoma) and Vero E6 cells (African green monkey kidney cells) were maintained in Dulbecco Modified Eagle Medium (DMEM; Invitrogen Life Technologies) supplemented with 10% heat-inactivated fetal bovine serum (FBS) and 1% penicillin/streptomycin (P/S) and 1% GlutaMAX (Life Technologies).

In cellulo imaging of the SARS-CoV-2 S-RBD/ACE2 interaction.

10 000 HEK293T and HEK293T-ACE2 plated on glass coverslips pretreated with poly-L-Lysine solution 0.01% (SIGMA ref P4832) 5 minutes at room temperature. After 24 hours the RBD recombinant protein (4-40 pmoles) was added to the cells in 100µl PBS. Cells were then washed and fixed (PFA 4%) at different time points. Cell imaging was performed as described above.

For quantification of the S-RBD/ACE2 interaction in cellular context 45 000 HEK293T and HEK293T-ACE2 cells were incubated 45 minutes at 37°C in 100 µl Dulbecco's Modified Eagle's Medium (DMEM) and increasing concentrations of RBD recombinant protein. One ml of PBS was added the cells were centrifugated 5 minutes at 2 500 rpm. 50 µl of anti-His antibody 1/200 in DMEM was added and the cells were further incubated 45 minutes at 37°C. After PBS washes 50 µl of a 1/400 DMEM solution of secondary antibody was added and cells were incubated 30 minutes at 37°C. After PBS washes the cells were resuspended in 200µl PBS, FBS 2% EDTA 2mM and the percentage of FITC positive cells was quantified by flow cytometry.

### Cytotoxicity assay

MTT cell viability assay was performed following furnisher protocols. Cells were seeded at the density of 20,000 cells/well in a 96 well plate containing 100 µL complete DMEM (Gibco, USA) supplemented with 10% FBS (Gibco, USA) and 1% Penstrep (Gibco, USA). Cells were incubated for 12 hours at 37° C in humidified 5% CO2 incubator for adherence. After 12-hour incubation, the media was replaced with fresh media, and cells were treated with compounds. Untreated cells were considered as negative control, DMSO treated cells were considered as the vehicles. After the treatment, cells were incubated at 37 ° C in humidified 5% CO2 incubator. 48-hour post-treatment, 20 µL of MTT substrate (5 mg/mL) was added in each well and incubated for 4 additional hours at 37° C in the dark. The media was then carefully removed, and 492nm absorbance was measured.

### SARS-CoV2 production

SARS-CoV-2 strain 220_95 (EPI_ISL_469284) was isolated from nasopharyngeal swab specimens collected at Service de Virologie (Hospital Saint Louis, Paris) and grown as previously described (Onodi et al. (2021) ([13])). Briefly, virus was propagated on Vero E6 in DMEM-2% (DMEM supplemented with 2% FBS, 1% P/S, 1% GlutaMAX, and 25 mM Hepes). viruses were purified through a 20% sucrose cushion by ultracentrifugation at 80,000xg for 2 hours at 4°C. Pellets were resuspended in HNE 1X (HEPES 25mM, NaCl 100mM EDTA 0.5mM) aliquoted and stored at -80°C. *SARS-CoV-2 infection assay*

A549-Ace2 and Calu3 cells grown in 12-well plates were challenged with SARS-CoV-2 at a MOI of 0.05 in the presence of the indicated drug or with DMSO as a control. After 3 hours, cells were washed once with PBS and incubated with fresh media. Compounds were maintained throughout the course of infection.

To assess productive viral replication, we quantified the infectious viral particles release during infection 24 hours post-infection by RT-qPCR. Viruses were first inactivated by incubating the supernatants v/v with 1% Triton X-100 (Sigma) in PBS for 30 min under agitation at RT. Yields of viral RNA were quantified by real-time qPCR by using SARS-CoV-2 specific primers targeting the E gene with the Luna^{®} Universal One-Step RT-qPCR Kit (New England Biolabs) in a LightCycler 480 thermocycler (Roche) according to the manufacturer's protocol. The number of viral genomes is expressed as PFU equivalent/ml and was calculated by performing a standard curve with a similarly treated supernatant from a viral stock with a known titer as described by Gordon et al. (Gordon et al. (2020) ([14])).

### Reference List

1. WO2008/055969.
2. Guillaume Bordeau: « Nouvelles tryarylamines pour la microscopie biphotonique : application au marquage des acides nucléiques », Thesis, 2009.
3. Bordeau,G., Lartia,R. and Teulade-Fichou,M.-P. (2010) meta-Substituted triphenylamines as new dyes displaying exceptionally large Stokes shifts. Tetrahedron Lett., 51, 4429-4432.
4. C. Allain et al., ChemBioChem 2007. C.Allain, F. Schmidt, R. Lartia, G. Bordeau, C. Fiorini-Debuischert , F. Charra, P. Tauc, M.-P. Teulade-Fichou Vinyl-pyridinium Triphenylamines : novel far-red emitters with high photostability and two-photon absorption properties for staining DNA ChemBioChem 2007,8, 424-433.
5. Lapaillerie,D., Charlier,C., Fernandes,H.S., Sousa,S.F., Lesbats,P., Weigel,P., Favereaux,A., Guyonnet-Duperat,V. and Parissi,V. (2021) In Silico, In Vitro and In Cellulo Models for Monitoring SARS-CoV-2 Spike/Human ACE2 Complex, Viral Entry and Cell Fusion. Viruses, 13.
6. Lan,J., Ge,J., Yu,J., Shan,S., Zhou,H., Fan,S., Zhang,Q., Shi,X., Wang,Q., Zhang,L., et al. (2020) Structure of the SARS-CoV-2 spike receptor-binding domain bound to the ACE2 receptor. Nature, 581, 215-220.
7. Crawford,K.H.D., Eguia,R., Dingens,A.S., Loes,A.N., Malone,K.D., Wolf,C.R., Chu,H.Y., Tortorici,M.A., Veesler,D., Murphy,M., et al. (2020) Protocol and Reagents for Pseudotyping Lentiviral Particles with SARS-CoV-2 Spike Protein for Neutralization Assays. Viruses, 12.
8. Shapovalov,M.V. and Dunbrack,R.L. (2011) A smoothed backbonedependent rotamer library for proteins derived from adaptive kernel density estimates and regressions. Struct. Lond. Engl. 1993, 19, 844-858.
9. Serrano,C., Teixeira,C.S.S., Cooper,D.N., Carneiro,J., Lopes-Marques,M., Stenson,P.D., Amorim,A., Prata,M.J., Sousa,S.F. and Azevedo,L. (2021) Compensatory epistasis explored by molecular dynamics simulations. Hum. Genet., 140, 1329-1342.
10.Improving the Catalytic Power of the DszD Enzyme for the Biodesulfurization of Crude Oil and Derivatives - Ferreira - 2017 - Chemistry - A European Journal - Wiley Online Library.
11.Adasme,M.F., Linnemann,K.L., Bolz,S.N., Kaiser,F., Salentin,S., Haupt,V.J. and Schroeder,M. (2021) PLIP 2021: expanding the scope of the protein-ligand interaction profiler to DNA and RNA. Nucleic Acids Res., 49, W530-W534.
12.Wrapp,D., Wang,N., Corbett,K.S., Goldsmith,J.A., Hsieh,C.-L., Abiona,O., Graham,B.S. and McLellan,J.S. (2020) Cryo-EM structure of the 2019-nCoV spike in the prefusion conformation. Science, 367, 1260-1263.
13.Onodi,F., Bonnet-Madin,L., Meertens,L., Karpf,L., Poirot,J., Zhang,S.-Y., Picard,C., Puel,A., Jouanguy,E., Zhang,Q., et al. (2021) SARS-CoV-2 induces human plasmacytoid predendritic cell diversification via UNC93B and IRAK4. J. Exp. Med., 218.
14.Gordon,D.E., Hiatt,J., Bouhaddou,M., Rezelj,V.V., Ulferts,S., Braberg,H., Jureka,A.S., Obernier,K., Guo,J.Z., Batra,J., et al. (2020) Comparative host-coronavirus protein interaction networks reveal pan-viral disease mechanisms. Science, 10.1126/science.abe9403.
15.Bordeau,G., Lartia,R. and Teulade-Fichou,M.-P. (2010) meta-Substituted triphenylamines as new dyes displaying exceptionally large Stokes shifts. Tetrahedron Lett., 51, 4429-4432.

## Claims

1. A pharmaceutical composition comprising a compound having formula (I) below: 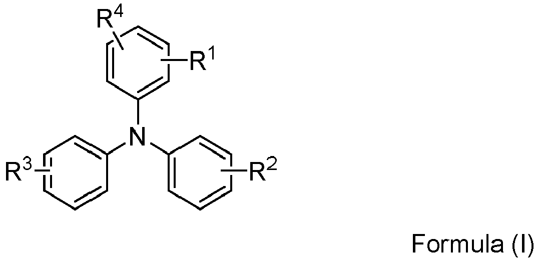 wherein:
- R¹ is chosen from the group comprising: 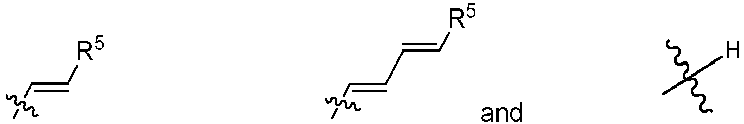
- R² is chosen from the group comprising: 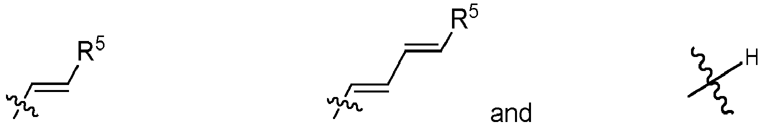
- R³ is chosen from the group comprising: 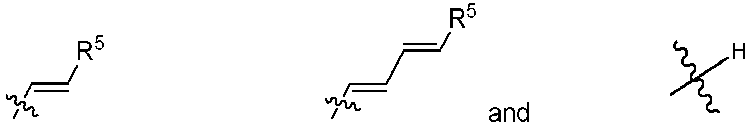 with R⁵ being chosen from the group comprising a heterocyclic moiety having 5 or 6 members and a heterocycle compound having 5 or 6 members condensed with a benzene ring,
- R⁴ is chosen from the group comprising a hydrogen, an alkyl or a PEG chain, terminated by a function chosen among OH, NH₂, COOH, OR and COOR.

2. The pharmaceutical composition according to claim 1, wherein :
R¹, R² and R³ are identical and are chosen from the group comprising:

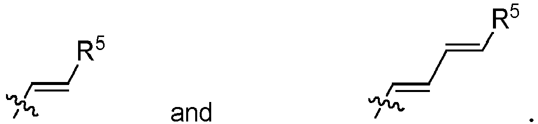

3. The pharmaceutical composition according to claim 1, wherein :
- R¹ and R² are identical and are chosen from the group comprising: 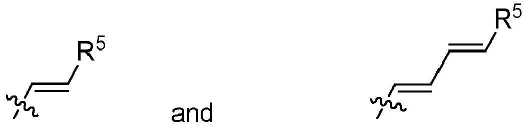
- R³ is

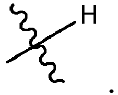

4. The pharmaceutical composition according to anyone of claims 1 to 3, wherein R¹, R² and R³ are linked in a symmetrical position on the Formula (I).

5. The pharmaceutical composition according to anyone of claims 1 to 3, wherein R¹, R² and R³ are linked in an asymmetrical position on the Formula (I).

6. The pharmaceutical composition according to claim 1, wherein :
- R¹ is chosen from the group comprising: 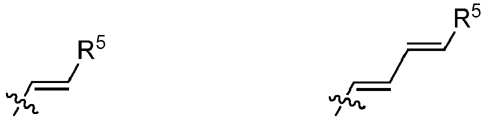
- R² and R³ are identical and are

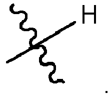

7. The pharmaceutical composition according to claim 1, wherein said compound is chosen among:

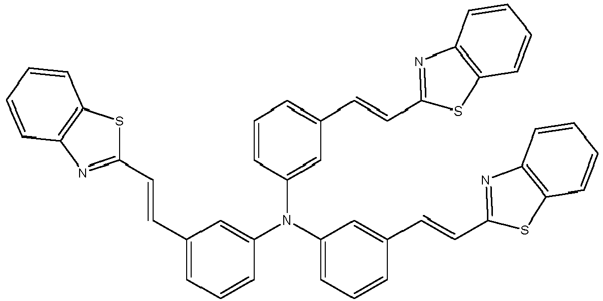

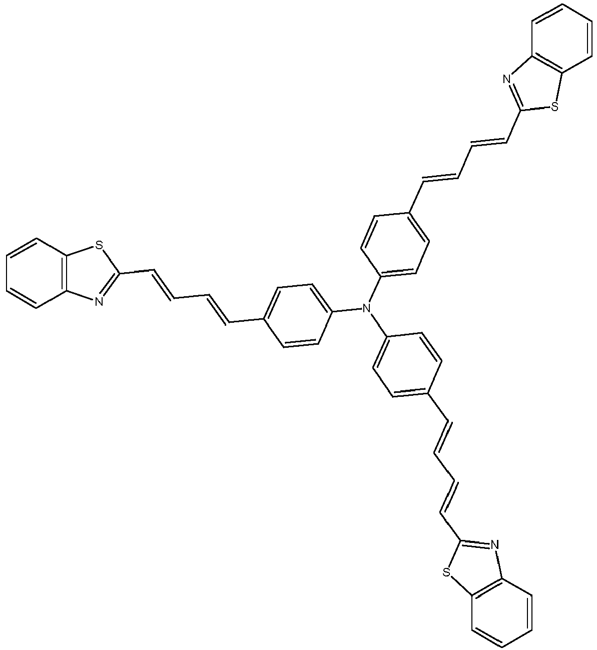

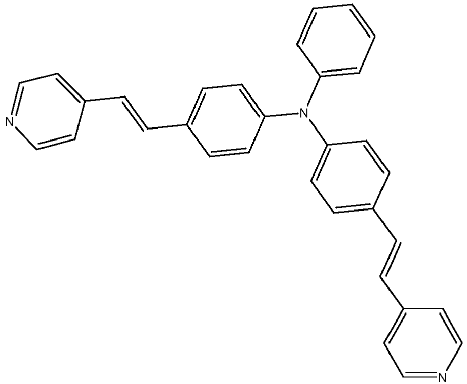

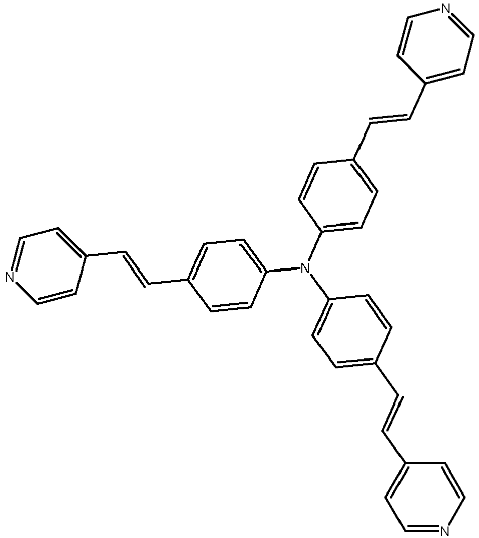

8. The pharmaceutical composition as claimed in anyone of the preceding claims, said pharmaceutical composition being in an oral or a parenteral form.

9. The pharmaceutical composition as claimed in anyone of the preceding claims, for use as a medicament.

10. The pharmaceutical composition as claimed in anyone of the preceding claims, for use in the prevention or the treatment of an infection.

11. The pharmaceutical composition for use as claimed in claim 10, for use in the prevention or the treatment of SARS-CoV-2 infection.

12. A compound as defined in anyone of claims 1 to 7, for use as a medicament.

13. A compound as defined in anyone of claims 1 to 7, for use in the prevention or the treatment of an infection.

14. A compound for use as claimed in claim 13, for use in the prevention or the treatment of SARS-CoV-2 infection.
